# EUROPÄISCHE PATENTSCHRIFT

(11) **EP 2 653 118 B1**
(45) Veröffentlichungstag und Bekanntmachung des Hinweises auf die Patenterteilung: **03.01.2018**
(21) Anmeldenummer: 13001768.4
(22) Anmeldetag: 06.04.2013
(51) Int. Cl.: A61B 17/29, A61B 17/295, A61B 18/14, A61B 17/00, A61B 18/00

(54) **Mikroinvasives medizinisches Instrument**
Micro-invasive medical instrument
Instrument médical micro-invasif

(30) Priorität: 18.04.2012 DE 102012007648
(43) Veröffentlichungstag der Anmeldung: 23.10.2013
(73) Patentinhaber: Karl Storz GmbH & Co. KG, 78532 Tuttlingen (DE)
(72) Erfinder: Bacher, Uwe, D-78532 Tuttlingen (DE); Kärcher, Daniel, D-78532 Tuttlingen (DE); Merz, Robin, D-78532 Tuttlingen (DE); Schneider, Sven, D-78532 Tuttlingen (DE); Stefan, Jochen, D-78532 Tuttlingen (DE)

(56) Entgegenhaltungen:
- EP-A1- 1 774 914
- EP-A2- 2 366 338
- WO-A2-95/08945
- US-A- 5 540 375
- US-A1- 2011 251 613
- US-A1- 2011 276 048
- US-A1- 2012 080 500

## Beschreibung

Die vorliegende Erfindung ist auf ein medizinisches Instrument für mikroinvasiv-chirurgische Anwendungen bezogen.

Die Erwartungen an medizinische Instrumente für mikroinvasive Eingriffe steigen beständig. Bereits in Vielfalt angeboten und weit verbreitet sind medizinische Instrumente mit einem Werkzeug mit Greif- oder Schneidefunktion am distalen Ende, bei denen das Werkzeug um die Längsachse des Schafts rotierbar ist. Die Greif- oder Schneidefunktion und die Rotation des Werkzeugs sind beispielsweise mittels einer einzigen Übertragungsstange steuerbar, die Längskräfte und Drehmomente überträgt. Hinzu kommt in neuerer Zeit eine Abwinkelbarkeit des Schafts proximal des Werkzeugs, zu deren Steuerung ein zweites Übertragungselement im Schaft des medizinischen Instruments vorgesehen sein kann, beispielsweise eine zweite Übertragungsstange.

Die Patentanmeldung US 2012/080500 A1 zeigt ein chirurgisches Schneide-und Klammergerät mit einem langerstreckten gelenkigen Schaft, der mit einer Griffanordnung gekoppelt ist. Dieses Dokument zeigt genau eine Klammer- und eine Schneidfunktion. Dieses Dokument zeigt auch Übertragungseinrichtungen für die Klammer- sowie die Schneidfunktion. Auch ist in US 2012/080500 A1 für das Schaft des Schneide-und Klammergeräts ein proximaler und ein distaler Abschnitt offenbart, die gelenkig miteinander verbunden sind, sowie eine Übertragungseinrichtung, die biegeflexibel ausgeführt ist. Das Dokument offenbart ein medizinisches Instrument dementsprechend dem Oberbegriff des unabhängigen Anspruchs 1. US20110251613A1 zeigt ein chirurgisches Instrument mit einem flexiblen Schaft, ein Werkzeug mit zwei Maulteilen und darüber hinaus ein Messer, das zwischen den Maulteilen axial bewegt werden kann. Eine Übertragungseinrichtung ist einseitig mit dem Handgriff verbunden, an ihrer anderen Seite mit dem Werkzeug, um eine Betätigung vom Handgriff auf das Werkzeug zu übertragen.
Die Druckschrift WO 95/08945 A2 offenbart ein chirurgisches Schneide-und Klammergerät mit einem langerstreckten biegsamen Schaft. Der Schaft ist mit einer Griffanordnung gekoppelt, und weist sowohl ein Greif- als auch ein Schneid-Werkzeug auf und für beide natürlich Übertragungseinrichtungen.
In US 5 540 375 A ist ein endoskopisches Instrument zum Klammersetzen und Entnehmen von Körpergewebe gezeigt. Dieses weist eine Griffanordnung sowie einen Schaft und einen Maulteil-Bereich als endoskopischen Abschnitt auf, der durch eine Kanüle einsetzbar ist.
Aus dem Dokument EP 2 366 338 A2 ist ein laparoskopischer Nadelhalter als Rohrschaftinstrument bekannt, an dessen distalem Ende Zangen-Maulteile angeordnet sind. Diese werden von einer Stange als Kraftübertragungseinrichtung, die durch den Schaft läuft, betätigt. Der Rohrschaft weist mehrere, unter anderem gebogene Abschnitte auf, die unter anderem mittels Bajonettverbindung miteinander verbunden werden können.
EP 1 774 914 A1 offenbart eine Vorrichtung, mit der chirurgische Befestigungselemente an einer Vielzahl von Körperstellen und -geweben sequentiellen gesetzt werden können.

Zudem kann gemäß dieser Patentanmeldung das Gewebe geschnitten werden. Die Vorrichtung umfasst einen länglichen Körper und eine Ladevorrichtung für Klammem. Dabei sind verschiedene Schaftabschnitte miteinander verbunden, u.a. mittels Bajonettverbindung.
Die Druckschrift US 2011/276048 A1 zeigt eine Zange mit einem Handgriff-Gehäuse, einem Schaft und einem Werkzeug mit zwei Klemmbacken zum Ergreifen von Gewebe. Zusätzlich ist hier ein Schneidelement mit einer Vielzahl von Schneidkanten gezeigt, die um eine Längsachse und innerhalb eines Schafts angeordnet und in Längsrichtung zwischen einer zurückgezogenen Position und einer ausgefahrenen Position verschiebbar sind. Das Schneidelement erstreckt sich dabei zwischen den Klemmbacken, um das dort erfasste Gewebe zu schneiden.
Eine Aufgabe der vorliegenden Erfindung besteht darin, ein verbessertes medizinisches Instrument zu schaffen, insbesondere ein medizinisches Instrument, das noch vielfältiger einsetzbar und an unterschiedliche Aufgaben und Anwendungen besser anpassbar ist.

Diese Aufgabe wird durch den Gegenstand des unabhängigen Anspruchs 1 gelöst. Weiterbildungen sind in den abhängigen Ansprüchen angegeben.
Ein medizinisches Instrument umfasst einen Außenschaft, eine Handhabungseinrichtung am proximalen Ende des Außenschafts, ein Werkzeug am distalen Ende des Außenschafts, wobei das Werkzeug eine erste Wirkeinrichtung für eine erste Funktion und eine zweite Wirkeinrichtung für eine zweite Funktion aufweist, eine erste Übertragungseinrichtung in dem Außenschaft zum Übertragen zumindest entweder einer Kraft oder eines Drehmoments zum Steuern der ersten Wirkeinrichtung und eine zweite Übertragungseinrichtung in dem Außenschaft zum Übertragen zumindest entweder einer Kraft oder eines Drehmoments zum Steuern der zweiten Wirkeinrichtung, wobei der Außenschaft zumindest entweder gekrümmt oder krümmbar ist oder ein Schwenkgelenk aufweist, und wobei die erste Übertragungseinrichtung und die zweite Übertragungseinrichtung jeweils zumindest abschnittsweise biegeflexibel ausgebildet sind.

Das medizinische Instrument ist insbesondere ein medizinisches Instrument für mikroinvasiv-chirurgische Anwendungen. Die zweite Wirkeinrichtung ist für eine zweite Funktion ausgebildet, die von der ersten Funktion verschieden ist und unabhängig von der ersten Funktion gesteuert werden kann. Dazu sind die erste Wirkeinrichtung mittels der ersten Übertragungseinrichtung und die zweite Wirkeinrichtung mittels der zweiten Übertragungseinrichtung unabhängig voneinander steuerbar.

Insbesondere weisen die erste Wirkeinrichtung und die zweite Wirkeinrichtung keine gemeinsamen Bestandteile auf. Die zweite Wirkeinrichtung kann als vollständig eigenständiges und von der ersten Wirkeinrichtung zerstörungsfrei trennbares Bauteil ausgebildet sein. Die erste Wirkeinrichtung ist insbesondere ausgebildet, um ihre vorgesehene Funktion und Wirkung auch in Abwesenheit der Schneideeinrichtung aufzuweisen bzw. ausüben zu können.

Die erste Wirkeinrichtung umfasst insbesondere mehrere Maulteile, von denen mindestens eines schwenkbar ist, zum Greifen oder Quetschen von Gewebe. Die zweite Wirkeinrichtung umfasst insbesondere ein Skalpell bzw. ein Messer mit einer Schneide zum Durchtrennen von Gewebe, insbesondere zum Durchtrennen von zuvor mittels der ersten Wirkeinrichtung gegriffenem, gequetschtem und verödetem Gewebe.

Der Außenschaft ist insbesondere gekrümmt, um eine Verwendung in der Single-Port-Chirurgie zu ermöglichen oder zu vereinfachen, bei der mehrere medizinische Instrumente durch eine einzige Öffnung bzw. einen einzigen Zugang in einen Hohlraum eingeführt werden. Mit einer Krümmbarkeit des Außenschafts ist eine reversible, zerstörungsfreie elastische und/oder plastische Verformbarkeit des Außenschafts gemeint. Alternativ oder zusätzlich zu einer Krümmung oder einer Krümmbarkeit des Außenschafts kann ein Schwenkgelenk mit einer Schwenkachse, die im Wesentlichen senkrecht zur Längsachse des Außenschafts ist, vorgesehen sein. Im Falle einer Krümmung des Außenschafts ist die Schwenkachse des Schwenkgelenks insbesondere senkrecht zur lokalen Längsachse des Außenschafts nahe dem Schwenkgelenk.

Insbesondere für die Single-Port-Chirurgie kann ein (beispielsweise im Wesentlichen schraubenförmig) gekrümmter Außenschaft mit einem Schwenkgelenk nahe seinem distalen Ende die Einsatzmöglichkeiten des Außenschafts deutlich verbessern.

Die erste Übertragungseinrichtung und die zweite Übertragungseinrichtung sind jeweils vollständig biegeflexibel oder weisen einen oder mehrere biegeflexible Abschnitte auf. Zur Übertragung von Längskräften und/oder Drehmomenten sind die Übertragungseinrichtungen jeweils in Längsrichtung steif und/oder torsionssteif ausgebildet. Die Biegeflexibilität der ersten Übertragungseinrichtung und der zweiten Übertragungseinrichtung beruht jeweils auf einer elastischen oder reversibel-plastischen Verformbarkeit, insbesondere auf Biegeelastizität, und/oder auf einem oder mehreren Schwenkgelenken in der entsprechenden Übertragungseinrichtung.

Ein medizinisches Instrument mit den hier beschriebenen Merkmalen kann eine besonders vielfältige bzw. universelle Verwendung ermöglichen. Das Werkzeug mit den beiden Wirkeinrichtungen für zwei verschiedene Funktionen kann aufgrund der Krümmung oder der Krümmbarkeit des Außenschafts und/oder mittels des Schwenkgelenks in besonders vielen Situation optimal zu dem Gewebe positioniert werden, das beispielsweise gegriffen, gequetscht, verödet und durchtrennt werden soll. Medizinisches Personal muss nicht mehr oder deutlich seltener entscheiden, welches medizinische Instrument aus einer Vielzahl alternativer Instrumente (mit unterschiedlichen Funktionen und unterschiedlicher Anordnung jeweils eines Werkzeugs mit einer einzigen Wirkeinrichtung) auszuwählen und zu verwenden ist. Eine größere Anzahl verschiedenerer Einzelschritte kann mit einem einzigen medizinischen Instrument durchführbar sein, so dass während eines mikroinvasivchirurgischen Eingriffs deutlich seltener das verwendete medizinische Instrument ausgetauscht werden muss. Dadurch können die für einen bestimmten mikroinvasivchirurgischen Eingriff erforderliche Zeitdauer, die Belastung für den Patienten und für das medizinische Personal sowie die Kosten gesenkt werden. Kostensenkend wirkt sich insbesondere aus, dass eine vielfältige Einsetzbarkeit des einzelnen medizinischen Instruments eine geringere Vielfalt und damit auch eine geringere Anzahl der bereitzuhaltenden Instrumente ermöglicht.

Bei einem medizinischen Instrument, wie es hier beschrieben ist, weisen Werkzeug und Außenschaft insbesondere Kupplungseinrichtungen zum lösbaren mechanischen Verbinden des Werkzeugs mit dem distalen Ende des Außenschafts auf.

Die Kupplungseinrichtungen sind insbesondere zur Bildung einer zerstörungsfrei und reversibel lösbaren mechanischen Verbindung ausgebildet. Die Kupplungseinrichtungen umfassen insbesondere Bajonett-Kupplungseinrichtungen, Schraubgewinde oder andere Einrichtungen zur formschlüssigen oder kraftschlüssigen lösbaren mechanischen Verbindung.

Die Lösbarkeit der Verbindung zwischen Werkzeug und Außenschaft kann die Reinigung des medizinischen Instruments nach einer Verwendung und in Vorbereitung auf eine weitere Verwendung vereinfachen. Ferner kann die lösbare mechanische Verbindung zwischen Werkzeug und Außenschaft einen Austausch des Werkzeugs oder des Außenschafts im Falle eines Defekts vereinfachen. Ferner können mehrere verschiedene Werkzeuge mit unterschiedlichen Merkmalen und/oder mehrere verschiedene Außenschäfte mit unterschiedlichen Merkmalen bereitgehalten und in der Art eines Baukastensystems verwendet werden.

Bei einem medizinischen Instrument, wie es hier beschrieben ist, weisen Außenschaft und Handhabungseinrichtung insbesondere Kupplungseinrichtungen zum lösbaren mechanischen Verbinden des proximalen Endes des Außenschafts mit der Handhabungseinrichtung auf.

Die Kupplungseinrichtungen sind insbesondere für eine zerstörungsfrei und reversibel lösbare mechanische Verbindung von Außenschaft und Handhabungseinrichtung ausgebildet.

Bei einem medizinischen Instrument, wie es hier beschrieben ist, sind die erste Übertragungseinrichtung und die zweite Übertragungseinrichtung insbesondere koaxial im Außenschaft angeordnet.

Eine koaxiale Anordnung der ersten Übertragungseinrichtung und der zweiten Übertragungseinrichtung im Außenschaft ist gegeben, wenn die erste Übertragungseinrichtung und die zweite Übertragungseinrichtung koaxial zueinander im Außenschaft angeordnet sind. Ferner können die erste Übertragungseinrichtung und die zweite Übertragungseinrichtung koaxial zum Außenschaft angeordnet sein. Eine koaxiale Anordnung kann insbesondere eine Rotierbarkeit des Außenschafts relativ zur Handhabungseinrichtung und/oder eine Rotierbarkeit des Werkzeugs relativ zum Außenschaft und/oder eine Rotierbarkeit einer Schwenkgelenks relativ zu einem proximalen Abschnitt des Außenschafts vereinfachen.

Bei einem medizinischen Instrument, wie es hier beschrieben ist, sind die erste Übertragungseinrichtung und die zweite Übertragungseinrichtung insbesondere nebeneinander im Außenschaft angeordnet.

Eine Anordnung der ersten Übertragungseinrichtung und der zweiten Übertragungseinrichtung nebeneinander kann hinsichtlich des erforderlichen Bauraums, insbesondere hinsichtlich der erforderlichen Querschnitte vorteilhaft sein. Insbesondere kann eine geringe Querschnittsfläche des Außenschafts erzielbar sein als bei einer koaxialen Anordnung.

Ein medizinisches Instrument mit einem Schwenkgelenk, wie es hier beschrieben ist, umfasst insbesondere ferner eine dritte Übertragungseinrichtung, deren distales Ende mit dem Schwenkgelenk gekoppelt ist, zum Steuern des Schwenkgelenks.

Die dritte Übertragungseinrichtung ist insbesondere mittels eines Pleuels oder mittels Gleitflächen unmittelbar oder mittelbar derart mit dem Schwenkgelenk gekoppelt, dass eine longitudinale Translation der dritten Übertragungseinrichtung parallel zum Außenschaft mit einem Schwenken des Werkzeugs um die Schwenkachse des Schwenkgelenks einhergeht. Die dritte Übertragungseinrichtung ist insbesondere im Falle eines gekrümmten oder krümmbaren Außenschafts biegeelastisch ausgebildet.

Ein medizinisches Instrument, wie es hier beschrieben ist, umfasst insbesondere ferner ein Rotationsgelenk proximal des Schwenkgelenks, zum Rotieren des Schwenkgelenks relativ zum proximalen Ende des Außenschafts, wobei die dritte Übertragungseinrichtung ferner zum Steuern des Rotationsgelenks ausgebildet ist.

Das Rotationsgelenk ist insbesondere unmittelbar proximal des Schwenkgelenks angeordnet, wobei der Abstand zwischen der Schwenkachse des Schwenkgelenks und dem Rotationsgelenk nur wenige (maximal 5 oder 10) Außendurchmesser des Außenschafts beträgt. Die Rotationsachse des Rotationsgelenks ist insbesondere parallel zur Längsachse des Außenschafts, im Falle einer Krümmung oder einer Krümmbarkeit des Außenschafts parallel zur lokalen Längsachse des Außenschafts nahe dem Rotationsgelenk. Ein Rotieren des Schwenkgelenks um die Rotationsachse des Rotationsgelenks geht auch mit einer Rotation der Schwenkachse des Schwenkgelenks einher.

Bei einem medizinischen Instrument, wie es hier beschrieben ist, ist die dritte Übertragungseinrichtung insbesondere koaxial in dem Außenschaft angeordnet.

Insbesondere weist die dritte Übertragungseinrichtung die Gestalt eines Rohres oder eines Schlauchs auf und ist in einem im Wesentlichen ringförmigen Zwischenraum zwischen dem Außenschaft einerseits und der ersten Übertragungseinrichtung und der zweiten Übertragungseinrichtung andererseits angeordnet.

Bei einem medizinischen Instrument, wie es hier beschrieben ist, ist die erste Wirkeinrichtung insbesondere als bipolar-elektrochirurgisches Instrument mit voneinander elektrisch isolierten Elektroden ausgebildet, wobei je eine der voneinander elektrisch isolierten Elektroden mit dem Außenschaft und mit der ersten Übertragungseinrichtung elektrisch leitfähig verbunden ist.

Die voneinander elektrisch isolierten Elektroden werden insbesondere durch jeweils ein Maulteil der ersten Wirkeinrichtung gebildet oder sind an jeweils einem Maulteil der ersten Wirkeinrichtung vorgesehen. Die voneinander elektrisch isolierten Elektroden sind über den Außenschaft bzw. über die erste Übertragungseinrichtung mit einer elektrischen Hochfrequenz-Leistungsquelle bzw. deren Polen verbindbar.

Bei einem medizinischen Instrument, wie es hier beschrieben ist, sind die zweite Übertragungseinrichtung mittels einer Bajonettkupplung mit dem Werkzeug lösbar mechanisch verbunden und an der zweiten Übertragungseinrichtung eine Verriegelungseinrichtung zur Kopplung der zweiten Übertragungseinrichtung mit der ersten Übertragungseinrichtung oder mit dem Außenschaft oder insbesondere mit einer dritten Übertragungseinrichtung derart, dass die zweite Übertragungseinrichtung relativ zu der ersten Übertragungseinrichtung oder relativ zu dem Außenschaft oder insbesondere relativ zu der dritten Übertragungseinrichtung nicht rotierbar ist, vorgesehen.

Die zweite Übertragungseinrichtung ist mittels der Bajonettkupplung insbesondere unmittelbar mit der zweiten Wirkeinrichtung verbunden. Die Verriegelungseinrichtung dient der mittelbaren Verriegelung der Bajonettkupplung dadurch, dass die zum Lösen der Bajonettkupplung erforderliche Rotation der zweiten Übertragungseinrichtung relativ zum Werkzeug, insbesondere relativ zur zweiten Wirkeinrichtung, unterbunden wird. Die Verriegelungseinrichtung kann nahe der Bajonettkupplung und somit nahe dem distalen Ende der zweiten Übertragungseinrichtung vorgesehen sein. Im Falle einer ausreichenden Torsionssteifigkeit der zweiten Übertragungseinrichtung sowie des Außenschafts, der ersten Übertragungseinrichtung oder der dritten Übertragungseinrichtung kann die Verriegelungseinrichtung alterativ an einem beliebigen anderen Ort an der zweiten Übertragungseinrichtung vorgesehen sein, beispielsweise an deren proximalem Ende.

Die beschriebene Kombination einer Bajonettkupplung mit einer Verriegelungseinrichtung, kann eine Lösbarkeit der mechanischen Verbindung von Werkzeug und zweiter Übertragungseinrichtung bei geringem Bauraumbedarf ermöglichen. Da die Verriegelungseinrichtung beabstandet von der Bajonettkupplung angeordnet sein kann, beispielsweise am proximalen Ende des medizinischen Instruments, kann insbesondere am distalen Ende des medizinischen Instruments Bauram eingespart werden.

Ein medizinisches Instrument, wie es hier beschrieben ist, umfasst insbesondere ferner ein Rotationsgelenk proximal des Werkzeugs, zum Rotieren des Werkzeugs relativ zum Außenschaft.

Das Rotationsgelenk proximal des Werkzeugs kann mit dem Werkzeug unlösbar verbunden sein. Im Falle eines Schwenkgelenks ist das Rotationsgelenk distal des Schwenkgelenks bzw. zwischen Werkzeug und Schwenkgelenk vorgesehen. Das Rotationsgelenk proximal des Werkzeugs kann eine optimale Ausrichtung bzw. Orientierung des Werkzeugs ermöglichen.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine weitere schematische Darstellung des medizinischen Instruments aus Figur 1;
- Figur 3: eine weitere schematische Darstellung des medizinischen Instruments aus den Figuren 1 und 2;
- Figur 4: eine weitere schematische Darstellung des medizinischen Instruments aus den Figuren 1 bis 3;
- Figur 5: eine schematische axonometrische Darstellung einer Greifeinrichtung für ein medizinisches Instrument;
- Figur 6: eine schematische axonometrische Darstellung einer Schneideeinrichtung;
- Figur 7: eine weitere schematische axonometrische Darstellung der Einrichtungen aus den Figur 5 und 6;
- Figur 8: eine schematische Schnittdarstellung einer Übertragungseinrichtung und eines Innenschafts;
- Figur 9: eine schematische Schnittdarstellung einer weiteren Übertragungseinrichtung und eines Innenschafts;
- Figur 10: eine weitere schematische Schnittdarstellung der Übertragungseinrichtung und des Innenschafts aus Figur 9.

### Beschreibung der Ausführungsformen

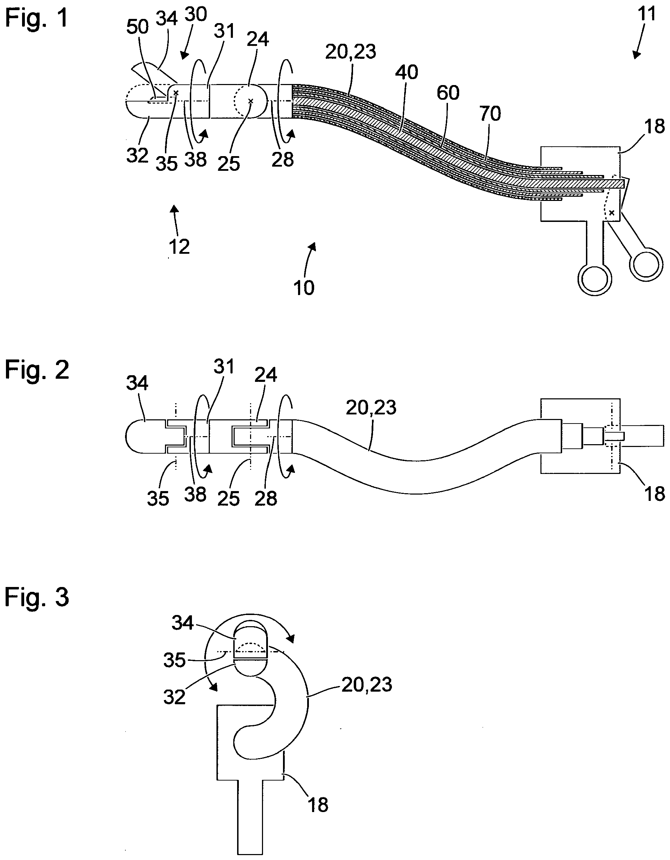

Figur 1 zeigt eine schematische Darstellung eines medizinischen Instruments 10 mit einem proximalen Ende 11 und einem distalen Ende 12. Das medizinische Instrument 10 weist am proximalen Ende 11 eine Handhabungseinrichtung 18 mit mehreren bewegbaren Griffteilen und anderen Betätigungseinrichtungen auf. Ein gekrümmter Außenschaft 20 erstreckt sich von der Handhabungseinrichtung 18 am proximalen Ende 11 bis zu einer Greifeinrichtung 30 und einer Schneideeinrichtung 50 am distalen Ende 12 des medizinischen Instruments 10.

Während in Figur 1 die Handhabungseinrichtung 18 lediglich durch ihre Umrisse angedeutet und die Greifeinrichtung 30 und die Schneideeinrichtung 50 in einer Seitenansicht gezeigt sind, ist ein im Wesentlichen rohrförmiger Abschnitt 23 des Außenschafts 20 im Längsschnitt dargestellt. Der rohrförmige Abschnitt 23 enthält in einer koaxialen Anordnung eine Übertragungsstange 40, einen inneren Innenschaft 60 und einen äußeren Innenschaft 70. Der innere Innenschaft 60 und der äußere Innenschaft 70 sind jeweils rohr- bzw. schlauchförmig ausgebildet. Die Übertragungsstange 40, der innere Innenschaft 60 und der äußere Innenschaft 70 sind jeweils biegeelastisch, hinsichtlich Längskräften steif und torsionssteif ausgebildet. Der innere Innenschaft 60 ist in einem ringförmigen Zwischenraum zwischen der Übertragungsstange 40 und dem äußeren Innenschaft 70 angeordnet. Der äußere Innenschaft 70 ist in einem ringförmigen Zwischenraum zwischen dem inneren Innenschaft 60 und dem rohrförmigen Abschnitt 23 des Außenschafts 20 angeordnet.

Unmittelbar proximal der Greifeinrichtung 30 ist ein Schwenkgelenk 24 vorgesehen, das mit dem rohrförmigen Abschnitt 23 des Außenschafts 20 unlösbar oder lösbar mechanisch verbunden ist und insbesondere einen Bestandteil des Außenschafts 20 bildet. Das Schwenkgelenk 24 ermöglicht ein Schwenken der Greifeinrichtung 30 und der Schneideeinrichtung 50 um eine Schwenkachse 25 senkrecht zur Zeichenebene der Figur 1. Das Schwenkgelenk 24 und die Schwenkachse 25 sind relativ zum gekrümmten, rohrförmigen Abschnitt 23 des Außenschafts 20 um eine Rotationsachse 28 in der Zeichenebene der Figur 1 rotierbar. Die Rotationsachse 28 ist durch ein in Figur 1 nicht dargestelltes Rotationsgelenk definiert.

Das proximale Ende 31 der Greifeinrichtung 30 ist mit dem Schwenkgelenk 24 lösbar mechanisch verbunden. Ein feststehendes Maulteil 32 und ein schwenkbares Maulteil 34, das um eine Schwenkachse 35 senkrecht zur Zeichenebene der Figur 1 schwenkbar ist, bilden das distale Ende der Greifeinrichtung 30. Das schwenkbare Maulteil 34 ist in durchgezogener Linie in einer offenen bzw. vom feststehenden Maulteil 32 beabstandeten Position und in gestrichelter Linie in einer geschlossenen bzw. am feststehenden Maulteil 32 anliegenden Position dargestellt. Die Greifeinrichtung 30 ist zusammen mit der Schwenkachse 35 und der Schneideeinrichtung 50 um die Längsachse 38 der Greifeinrichtung 30 relativ zum Schwenkgelenk 24 rotierbar.

Figur 2 zeigt eine weitere schematische Darstellung des medizinischen Instruments 10 aus Figur 1. Die Zeichenebene der Figur 2 ist senkrecht zur Zeichenebene der Figur 1 und parallel zur Schwenkachse 25 des Schwenkgelenks 24 und zur Rotationsachse 28, um die das Schwenkgelenk 24 relativ zum rohrförmigen Abschnitt 23 des Außenschafts 20 rotierbar ist. Anders als in Figur 1 ist der Außenschaft 20 in Figur 2 ausschließlich in einer Außen- bzw. Seitenansicht dargestellt.

Figur 3 zeigt eine weitere schematische Darstellung des medizinischen Instruments aus den Figuren 1 und 2. Die Zeichenebene der Figur 3 ist senkrecht zur Zeichenebene der Figur 1, senkrecht zur Zeichenebene der Figur 2, senkrecht zur Längsachse 38 der Greifeinrichtung 30 (vgl. Figur 2), senkrecht zur Rotationsachse 28, um die das Schwenkgelenk 24 relativ zum rohrförmigen Abschnitt 23 des Außenschafts 20 rotierbar ist, parallel zur Schwenkachse 25 des Schwenkgelenks 24 und parallel zur Schwenkachse 35 des schwenkbaren Maulteils 34.

In der Zusammenschau der Figuren 1 bis 3 ist erkennbar, dass der Außenschaft 20 räumlich bzw. nicht-eben gekrümmt ist, insbesondere eine einer Schraube bzw. einer Helix ähnliche Gestalt aufweist.

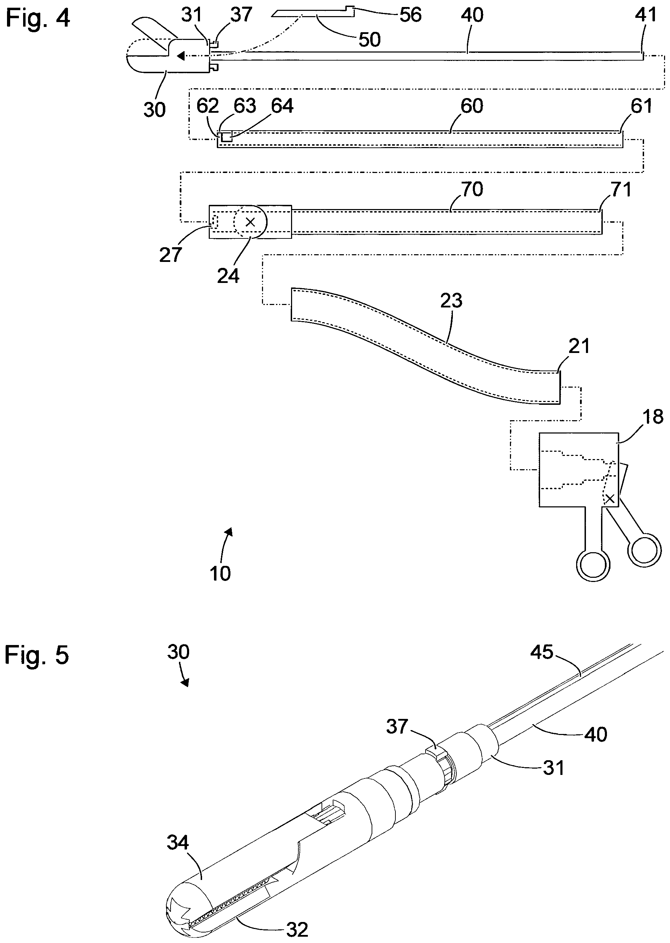

Figur 4 zeigt eine weitere schematische Darstellung des medizinischen Instruments aus den Figuren 1 bis 3. Die Zeichenebene der Figur 4 entspricht der Zeichenebene der Figur 1. Die Darstellung in Figur 4 unterscheidet sich von den Darstellungen in den Figuren 1 bis 3 dadurch, dass Bestandteile, in die das medizinische Instrument 10 durch medizinisches Personal ohne Verwendung von Werkzeug zerstörungsfrei bzw. reversibel zerlegbar ist, separat dargestellt sind. Die biegeelastische Übertragungsstange 40, der biegeelastische innere Innenschaft 60 und der biegeelastische äußere Innenschaft 70 sind jeweils gerade dargestellt. Strichpunktierte Linien deuten an, wie die Bestandteile des medizinischen Instruments zusammenzusetzen sind.

Die biegeelastische, torsionssteife und hinsichtlich Längskräften steife Übertragungstange 40 ist mit der Greifeinrichtung 30 mechanisch verbunden. Wie unten anhand der Figur 5 im Detail dargestellt ist, ist das distale Ende der Übertragungsstange 40 mit dem schwenkbaren Maulteil 34 mechanisch gekoppelt und elektrisch leitfähig verbunden und vom feststehenden Maulteil 32 und von Bajonettklauen bzw. Knaggen 37 am proximalen Ende 31 der Greifeinrichtung 30 elektrisch isoliert. Wie ebenfalls unten anhand der Figuren 5 bis 7 dargestellt ist, kann die Greifeinrichtung 50 von proximal in einen Kanal in der Übertragungsstange 40 und in die Greifeinrichtung 30 eingesetzt werden.

Der rohr- bzw. schlauchförmige innere Innenschaft 60 weist ein Lumen auf, dessen Querschnitt an den Querschnitt der Übertragungsstange 40 angepasst ist, so dass die Übertragungsstange 40 spiel- und reibungsarm in dem inneren Innenschaft 60 geführt ist. Am distalen Ende 62 weist der innere Innenschaft 60 einen L-förmigen Schlitz mit einem axialen Abschnitt 63 und einem umfänglichen Abschnitt 64 auf. Der L-förmige Schlitz 63, 64, ein Vorsprung 56 an der Schneideeinrichtung 50 und deren Funktion sind ebenfalls unten mit Bezug auf die Figuren 5 bis 7 dargestellt.

Der äußere Innenschaft 70 ist mit dem Schwenkgelenk 24 mechanisch verbunden. Insbesondere ist das distale Ende des äußeren Innenschafts 70 derart mit dem Schwenkgelenk 24 gekoppelt, dass eine axiale Translationsbewegung des äußeren Innenschafts 70 mit einer Schwenkbewegung des distalen Teils des Schwenkgelenks 24 um die Schwenkachse 25 relativ zum proximalen Teil des Schwenkgelenks 24 einhergeht. Am distalen Ende des Schwenkgelenks 24 sind L-förmige Nuten 27, die zu den Knaggen 37 am proximalen Ende 31 der Greifeinrichtung 30 korrespondieren, vorgesehen, zur lösbaren mechanischen Verbindung des Werkzeugs 30 mit dem Schwenkgelenkt 24. Das proximale Ende des Schwenkgelenks 24 ist mit dem distalen Ende des rohrförmigen Abschnitts 23 des Außenschafts 20 mittels Einrichtungen, die in Figur 4 nicht gezeigt sind, lösbar mechanisch verbindbar. Insbesondere sind am proximalen Ende des Schwenkgelenks 24 und am distalen Ende des rohrförmigen Abschnitts 23 des Außenschafts 20 Bajonett-Kupplungseinrichtungen vorgesehen.

Der äußere Innenschaft 70 ist im Wesentlichen rohr- bzw. schlauchförmig. Der Querschnitt des Lumens des äußeren Innenschafts 70 ist derart an den Querschnitt des inneren Innenschafts 60 angepasst, dass der innere Innenschaft 60 spiel- und reibungsarm im äußeren Innenschaft 70 geführt und bewegbar ist. Der Querschnitt des Lumens des rohrförmigen Abschnitts 23 des Außenschafts 20 ist derart an den Querschnitt des äußeren Innenschafts 70 angepasst, dass der äußere Innenschaft 70 spiel- und reibungsarm im rohrförmigen Abschnitt 23 des Außenschafts 20 geführt und bewegbar ist.

Das proximale Ende 21 des Außenschafts 20 bzw. des rohrförmigen Abschnitts 23 des Außenschafts 20 ist in einer korrespondierenden Ausnehmung in der Handhabungseinrichtung 18 mittels einer in den Figuren 1 bis 4 nicht dargestellten Verriegelungseinrichtung formschlüssig verriegelbar. Das proximale Ende 41 der Übertragungsstange 40, das proximale Ende 61 des inneren Innenschafts 60 und das proximale Ende 71 des äußeren Innenschafts 70 sind bei der in Figur 1 angedeuteten Anordnung mittels Einrichtungen, die in den Figuren 1 bis 4 nicht gezeigt sind, mit Betätigungseinrichtungen an der Handhabungseinrichtung 18 mechanisch gekoppelt.

Insbesondere ist das proximale Ende 41 der Übertragungsstange 40 mit einem schwenkbaren Griffteil der Handhabungseinrichtung 18 derart gekoppelt, dass die Übertragungsstange 40 in ihrer Längsrichtung verschiebbar ist, um das schwenkbare Maulteil 34 zwischen der in den Figuren 1, 3 und 4 in durchgezogenen Linien dargestellten offenen Position und der in den Figuren 1, 3 und 4 gestrichelten Linien dargestellten geschlossenen Position zu schwenken. Ferner ist die Übertragungsstange 40 mit einer weiteren Betätigungseinrichtung, beispielsweise einem Drehrad, derart gekoppelt, dass die Übertragungsstange 40 um ihre Längsachse rotierbar ist, um die Greifeinrichtung 30 zusammen mit der Schneideeinrichtung 50 um ihre Längsachse 38 zu rotieren.

Ferner ist das proximale Ende 61 des inneren Innenschafts 60 derart mit einer weiteren Betätigungseinrichtung der Handhabungseinrichtung 18 gekoppelt, dass der innere Innenschaft 60 axial bzw. in seiner Längsrichtung verschoben werden kann, um die Schneideeinrichtung 50 parallel zur Längsachse 38 der Greifeinrichtung 30 zu bewegen. Ferner ist das proximale Ende 71 des äußeren Innenschafts 70 derart mit einer weiteren Betätigungseinrichtung der Handhabungseinrichtung 18 mechanisch gekoppelt, dass der äußere Innenschaft 70 in axialer bzw. Längsrichtung verschiebbar ist, um die Greifeinrichtung 30 und die Schneideeinrichtung 50 zusammen mit dem distalen Teil des Schwenkgelenks 24 um die Schwenkachse 25 zu schwenken. Ferner ist das proximale Ende 71 des äußeren Innenschafts 70 derart mit einer weiteren Betätigungseinrichtung der Handhabungseinrichtung 18 mechanisch gekoppelt, dass der äußere Innenschaft um seine Längsachse rotiert werden kann, um das Schwenkgelenk 24 zusammen mit der Schwenkachse 25 um die Rotationsachse 28 relativ zum distalen Ende des rohrförmigen Abschnitts 23 des Außenschafts 20 zu rotieren.

Figur 5 zeigt eine schematische axonometrische Darstellung der Greifeinrichtung 30, die vorgesehen und ausgebildet ist zur Bildung eines medizinischen Instruments, wie es oben anhand der Figuren 1 bis 4 dargestellt ist. Die Greifeinrichtung 30 weist ein proximales Ende 31 und zwei Maulteile 32, 34, die das distale Ende der Greifeinrichtung 30 bilden, auf. Nahe dem proximalen Ende 31 weist die Greifeinrichtung 30 zwei symmetrisch angeordnete Bajonettklauen bzw. Knaggen 37 auf, von denen eine an einer vom Betrachter abgewandten Seite angeordnet und deshalb weitgehend verdeckt ist. Die in Figur 5 dargestellte Greifeinrichtung 30 unterscheidet sich von der oben anhand der Figuren 1 bis 4 dargestellten durch eine etwas andere Anordnung der Knaggen 37. Im Übrigen entspricht die in Figur 5 dargestellte Greifeinrichtung 30 im Wesentlichen der Greifeinrichtung des oben anhand der Figuren 1 bis 4 dargestellten medizinischen Instruments.

Die Greifeinrichtung 30 ist mit der Übertragungsstange 40 mechanisch verbunden. Die Übertragungsstange 40 ist relativ zur Greifeinrichtung 30, insbesondere relativ zum proximalen Ende 31 und zum feststehenden Maulteil 32 in axialer Richtung, d.h. parallel zur Längsachse der Übertragungsstange 40 und zur Längsachse 38 der Greifeinrichtung (vgl. Figuren 1 und 2) innerhalb eines vorbestimmten Intervalls bewegbar. Das innerhalb der Greifeinrichtung 30 angeordnete und deshalb in Figur 5 nicht sichtbare distale Ende der Übertragungsstange 40 ist mit dem schwenkbaren Maulteil 34 derart gekoppelt, dass eine axiale Bewegung der Übertragungsstange 40 mit einer Schwenkbewegung des schwenkbaren Maulteils 34 einhergeht.

In der Übertragungsstange 40 ist eine Nut 45 vorgesehen, die insbesondere einen schmalen und tiefen rechteckigen Querschnitt aufweist. Die Nut 45 in der Übertragungsstange 40 ist an deren in Figur 5 nicht sichtbaren distalen Ende durch einen Kanal entsprechenden Querschnitts fortgesetzt, der sich zwischen den Maulteilen 32, 34 bis fast zu deren distalen Enden erstreckt.

Teile der Greifeinrichtung 30, insbesondere die Knaggen 37 sowie die Übertragungsstange 40 sind aus Edelstahl oder einem anderen Metall gefertigt. Die Knaggen 37 und die Übertragungsstange 40 sind voneinander elektrisch isoliert. Die Maulteile 32, 34 weisen metallische und damit elektrisch leitfähige Greifflächen auf, die voneinander elektrisch isoliert sind, wenn sie nicht, wie in Figur 5 gezeigt, aneinander anliegen. Die Knaggen 37 und die Übertragungsstange 40 sind jeweils mit der Greiffläche eines Maulteils 32, 34 elektrisch leitfähig verbunden. Insbesondere sind die Knaggen 37 mit der Greiffläche des feststehenden Maulteils 32 und die Übertragungsstange 40 mit der Greiffläche des schwenkbaren Maulteils 34 elektrisch leitfähig verbunden.

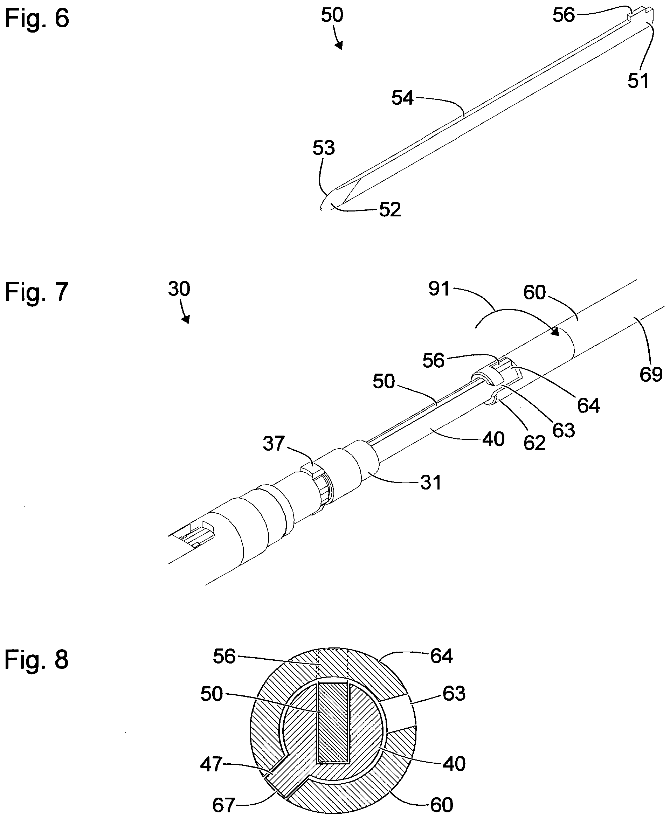

Figur 6 zeigt eine schematische axonometrische Darstellung der Schneideeinrichtung 50 mit einem proximalen Ende 51 und einem distalen Ende 52. Am distalen Ende 52 weist die Schneideeinrichtung 50 eine Schneide 53 auf. Am proximalen Ende weist die Schneideeinrichtung 50 einen Vorsprung 56 auf. Zwischen dem proximalen Ende 51 und dem distalen Ende 52 umfasst die Schneideeinrichtung einen stabförmigen Bereich 54, der im Wesentlichen die Gestalt einer streifenförmigen Platte bzw. eines Stabs mit rechteckigem Querschnitt aufweist.

Zwischen dem Vorsprung 56 am proximalen Ende 51 und der Schneide 53 am distalen Ende 52 entspricht der Querschnitt der Schneideeinrichtung 50 im Wesentlichen dem Querschnitt der Nut 45 in der Übertragungsstange 40 (vgl. Figur 5), so dass die Schneideeinrichtung 50 von dem Vorsprung 56 abgesehen, vollständig von der Nut 45 in der Übertragungsstange 40 aufgenommen und in dieser spiel- und reibungsarm geführt und in Längsrichtung der Übertragungsstange 40 und der Schneideeinrichtung 50 verschiebbar sein kann. Der Vorsprung 56 ist vorgesehen, um aus der Nut 45 in der Übertragungsstange 40 herauszuragen.

Figur 7 zeigt eine weitere schematische axonometrische Darstellung der Greifeinrichtung aus Figur 5 und der Schneideeinrichtung 50 aus Figur 6. Bei der Darstellung in Figur 7 ist die Schneideeinrichtung 50 in der Nut 45 in der Übertragungsstange 40 (vgl. Figur 5) angeordnet. Das distale Ende 52 der Schneideeinrichtung 50 (vgl. Figur 6) ist dabei in der Greifeinrichtung und insbesondere zwischen den Maulteilen 32, 34 angeordnet. Der Vorsprung 56 ragt aus der Nut 45 heraus.

In Figur 7 ist ferner der innere Innenschaft 60 gezeigt, der im Wesentlichen eine rohrförmige bzw. kreiszylindermantelförmige Gestalt aufweist. Der innere Innenschaft 60 weist an seinem distalen Ende 62 einen L-förmigen Schlitz mit einem axialen bzw. sich in axialer Richtung erstreckenden Abschnitt 63 und einem umfänglichen bzw. in umfänglicher Richtung sich erstreckenden Abschnitt 64 auf. Die in umfänglicher Richtung zu messende Breite des axialen Abschnitts 63 und die in axialer Richtung zu messende Breite des umfänglichen Abschnitts 64 des L-förmigen Schlitzes sind an die Abmessungen des Vorsprungs 56 an der Schneideeinrichtung 50 angepasst.

Nach einem Einführen der Übertragungsstange 40 in den inneren Innenschaft 60 kann der Vorsprung 56 durch eine Relativbewegung in axialer Richtung durch den axialen Abschnitt 63 bis in den umfänglichen Abschnitt 64 eingeführt werden. Wenn der Vorsprung 56 an der Schneideeinrichtung 50 sich im umfänglichen Abschnitt 64 des L-förmigen Schlitzes am distalen Ende 62 des inneren Innenschafts 60 befindet, kann der innere Innenschaft 60 relativ zu der Greifeinrichtung 30, der Übertragungsstange 40 und der Schneideeinrichtung 50 in einer ersten Richtung 91 bis zu der in Figur 7 gezeigten Konfiguration rotiert werden.

In der in Figur 7 gezeigten relativen Positionierung von Schneideeinrichtung 50 und innere Innenschaft 60 sind Schneideeinrichtung 50 und innere Innenschaft 60 hinsichtlich axialer Bewegungen miteinander (von Spiel abgesehen) starr gekoppelt. Eine axiale Bewegung des inneren Innenschafts 60 geht deshalb mit einer entsprechenden axialen Bewegung der Schneideeinrichtung 50 einher. Somit kann mittels des inneren Innenschafts 60 eine Bewegung der Schneide 53 am distalen Ende 52 der Schneideeinrichtung 50 (vgl. Figur 6) in dem erwähnten, in den Figuren nicht sichtbaren Kanal zwischen den Maulteilen 32, 34 bewirkt werden, um beispielsweise von den Maulteilen 32, 34 gegriffenes Gewebe nach einer Elektrokauterisation zu durchtrennen.

Der innere Innenschaft 60 weist einen Isoliermantel 69 auf, dessen distaler Rand nahe dem L-förmigen Schlitz 63, 64 liegt, und der sich bis nahe des proximalen Endes des inneren Innenschafts 60 erstrecken kann.

Figur 8 zeigt eine schematische Schnittdarstellung eines Beispiels für eine Verrieglung der oben anhand der Figur 7 dargestellten mechanischen Kopplung zwischen dem Vorsprung 56 am proximalen Ende der Schneideeinrichtung 50 und dem L-förmigen Schlitz 63, 64 am distalen Ende 62 des inneren Innenschafts 60. Die Schnittebene der Figur 8 ist senkrecht zur Zeichenebene der Figur 1, senkrecht zur Zeichenebene der Figur 2, parallel zur Zeichenebene der Figur 3 und senkrecht zur Zeichenebene der Figur 4. Die Schnittebene der Figur 8 liegt unmittelbar proximal des distalen Endes 62 des inneren Innenschafts 60 im Bereich des axialen Abschnitts 63 des L-förmigen Schlitzes (vgl. Figuren 4 und 7).

Der innere Innenschaft 60 weist einen axialen bzw. in axialer Richtung sich erstreckenden Verriegelungsschlitz 67 auf, der dem L-förmigen Schlitz 63, 64 im Wesentlichen gegenüber liegt. Der axiale Verriegelungsschlitz 67 liegt somit bezogen auf die Darstellungen in den Figuren 5 und 7 an einer vom Betrachter abgewandten Seite. Die Übertragungsstange 40 weist einen zu dem axialen Verriegelungsschlitz 67 im inneren Innenschaft 60 korrespondierenden Vorsprung 47 auf, beispielsweise in Form einer Nase, eines in axialer Richtung sich erstreckenden Stegs oder eines Zapfens.

Die Winkelposition des Vorsprungs 47 relativ zur Nut 45 (vgl. Figuren 4 und 7) in der Übertragungsstange 40 und die Winkelposition des axialen Verriegelungsschlitzes 67 relativ zum L-förmigen Schlitz 63, 64 im inneren Innenschaft 60 sind so gewählt, dass der Vorsprung 56 an der in der Nut 45 in der Übertragungsstange 40 angeordneten Schneideeinrichtung 50 im umfänglichen Abschnitt 64 des L-förmigen Schlitzes am distalen Ende 62 des inneren Innenschafts 60 gehalten ist, wenn der Vorsprung 47 an der Übertragungsstange 40 in den axialen Verriegelungsschlitz 67 am inneren Innenschaft 60, wie in Figur 8 dargestellt, eingreift. Der Vorsprung 47 ist soweit distal des proximalen Endes der Nut 45 in der Übertragungsstange 40 (vgl. Figuren 5 und 7) angeordnet, dass zunächst die Schneideeinrichtung 50 in die Nut 45 in der Übertragungsstange 40 eingesetzt und in der oben anhand der Figur 7 beschriebenen Weise mit dem distalen Ende 62 des inneren Innenschafts 60 gekoppelt werden kann. Danach wird der innere Innenschaft 60 zusammen mit der Schneideeinrichtung 50 nach distal verschoben. Erst bei dieser Verschiebung des inneren Innenschafts 60 nach distal greift der Vorsprung 47 an der Übertragungsstange 40 in den axialen Verriegelungsschlitz 67 am inneren Innenschaft 60 wie in Figur 8 dargestellt ein, um die mechanische Verbindung zwischen der Schneideeinrichtung 50 und dem inneren Innenschaft 60 zu verriegeln.

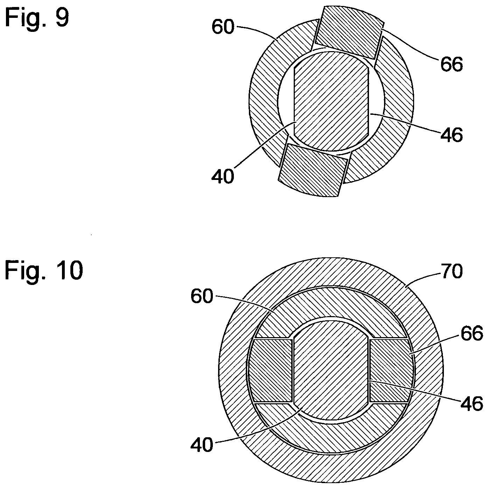

Figur 9 zeigt eine schematische Schnittdarstellung eines weiteren Beispiels einer Verriegelung des inneren Innenschafts 60 relativ zur Übertragungsstange40. Die Schnittebene der Figur 9 ist senkrecht zur Zeichenebene der Figur 1, senkrecht zur Zeichenebene der Figur 2, parallel zur Zeichenebene der Figur 3, senkrecht zur Zeichenebene der Figur 4 und parallel zur Schnittebene der Figur 8.

Die Übertragungsstange 40 weist zwei einander gegenüberliegende Abflachungen 46 auf. Der innere Innenschaft 60 weist zwei gegenüberliegende Öffnungen auf, in denen je ein Riegel 66 radial verschiebbar gelagert ist. Die Riegel 66 sind insbesondere zwischen radial innenliegenden verriegelnden Positionen und den in Figur 9 gezeigten radial außenliegenden entriegelnden Positionen verschiebbar. Die Riegel 66 können durch einen O-Ring aus einem elastischen Material oder durch anderen in Figur 9 nicht gezeigte Einrichtungen in den Ausnehmungen im inneren Innenschaft 60 gehalten und/oder nach radial innen zu den verriegelnden Positionen gedrückt werden.

Figur 10 zeigt eine weitere schematische Schnittdarstellung des Beispiels aus Figur 9. Die Schnittebene der Figur 10 entspricht der Schnittebene der Figur 9. Die Darstellung in Figur 10 unterscheidet sich von der Darstellung in Figur 9 dadurch, dass der innere Innenschaft 60 relativ zur Übertragungsstange 40 so weit rotiert ist, dass die Riegel 66 ihre radial innenliegenden verriegelnden Positionen einnehmen und dabei an den Abflachungen 46 an der Übertragungsstange 40 anliegen können.

Bei der Darstellung in Figur 10 sind die Übertragungsstange 40 und der innere Innenschaft 60 im äußeren Innenschaft 70 angeordnet. Dabei liegen radial äußere Oberflächen der Riegel 66 an der inneren Oberfläche des äußeren Innenschafts 70 an. Dadurch sind die Riegel 66 formschlüssig in ihrer in Figur 10 gezeigten verriegelnden Position gehalten. Somit ist bei der in Figur 10 gezeigten Anordnung der Übertragungsstange 40 und des inneren Innenschafts 60 im äußeren Innenschaft 70 die Übertragungsstange 40 hinsichtlich einer Rotation um ihre Längsachse relativ zum inneren Innenschaft 60 verriegelt.

Die Winkelpositionen der Abflachungen 46 relativ zu der Nut 45 in der Übertragungsstange 40 (vgl. Figuren 5 und 7) und die Winkelpositionen der die Riegel 66 aufnehmenden Öffnungen im inneren Innenschaft 60 relativ zum L-förmigen Schlitz 63, 64 am distalen Ende 62 des inneren Innenschafts 60 sind so gewählt, dass bei der in Figur 10 gezeigten verriegelten Konfiguration der Vorsprung 56 der Schneideeinrichtung 50 in der Nut 45 in der in Figur 7 gezeigten formschlüssigen Verbindung mit dem umfänglichen Abschnitt 64 des L-förmigen Schlitzes am distalen Ende 62 des inneren Innenschafts 60 gehalten ist. Somit ist in der in Figur 10 gezeigten Konfiguration die mechanische Verbindung zwischen Schneideeinrichtung 50 und Innenschaft 60 verriegelt.

Die anhand der Figuren 9 und 10 dargestellte Verriegelung zwischen Übertragungsstange 40 und innerem Innenschaft 60 kann nahe dem distalen Ende des inneren Innenschafts 60 oder, bei hinreichend torsionssteifer Ausführung der Übertragungsstange 40 und des inneren Innenschafts 60, an jedem beliebigen anderen Ort bis zu den proximalen Enden der Übertragungsstange 40 und des inneren Innenschafts 60 angeordnet sein.

### Bezugszeichen

- **10**: **medizinisches Instrument**
- 11: proximales Ende des medizinischen Instruments 10
- 12: distales Ende des medizinischen Instruments 10
- 18: Handhabungseinrichtung am proximalen Ende 11 des medizinischen Instruments 10
- **20**: **Außenschaft** des medizinischen Instruments 10
- 21: proximales Ende des Außenschafts 20
- 22: distales Ende des Außenschafts 20
- 23: rohrförmiger Abschnitt des Außenschafts 20
- 24: Schwenkgelenk am distalen Ende 22 des Außenschafts 20
- 25: Schwenkachse des Schwenkgelenks 24
- 27: L-förmige Nut am distalen Ende 22 des Außenschafts 20
- 28: Rotationsachse des Schwenkgelenks 24
- **30**: **Greifeinrichtung** am distalen Ende 12 des medizinischen Instruments 10
- 31: proximales Ende der Greifeinrichtung 30
- 32: feststehendes Maulteil der Greifeinrichtung 30
- 34: schwenkbares Maulteil der Greifeinrichtung 30
- 35: Schwenkachse des schwenkbaren Maulteils 34
- 37: Knagge am proximalen Ende 31 der Greifeinrichtung 30
- 38: Längsachse der Greifeinrichtung 30
- **40**: **Übertragungsstange** des medizinischen Instruments 10
- 41: proximales Ende der Übertragungsstange 40
- 45: Nut in der Übertragungsstange 40
- 46: Abflachung an der Übertragungsstange 40
- 47: Vorsprung an der Übertragungsstange 40
- **50**: **Schneideeinrichtung** am distalen Ende 12 des medizinischen Instruments 10
- 51: proximales Ende der Schneideeinrichtung 50
- 52: distales Ende der Schneideeinrichtung 50
- 53: Schneide an der Schneideeinrichtung 50
- 54: stabförmiger Bereich der Schneideeinrichtung 50
- 56: Vorsprung am proximalen Ende 51 der Schneideeinrichtung 50
- **60**: **innerer Innenschaft** des medizinischen Instruments 10
- 61: proximales Ende des inneren Innenschafts 60
- 62: distales Ende des inneren Innenschafts 60
- 63: axialer Abschnitt eines L-förmigen Schlitzes am distalen Ende 62
- 64: umfänglicher Abschnitt eines L-förmigen Schlitzes am distalen Ende 62
- 66: Riegel am inneren Innenschaft 60
- 67: axialer Verriegelungsschlitz am inneren Innenschaft 60
- 69: Isoliermantel am inneren Innenschaft 60
- **70**: **äußerer Innenschaft des medizinischen Instruments 10**
- 71: proximales Ende des äußeren Innenschafts 70

## Patentansprüche

1. **Medizinisches Instrument** (10), mit:
einem **Außenschaft** (20);
einer **Handhabungseinrichtung** (18) am proximalen Ende (21) des Außenschafts (20);
einem **Werkzeug** (30, 50) am distalen Ende des Außenschafts (20), mit einer ersten Wirkeinrichtung (30) für eine erste Funktion und einer zweiten Wirkeinrichtung (50) für eine zweite Funktion;
einer **ersten Übertragungseinrichtung** (40) in dem Außenschaft (20) zum Übertragen zumindest entweder einer Kraft oder eines Drehmoments zum Steuern der ersten Wirkeinrichtung (30);
einer **zweiten Übertragungseinrichtung** (60) in dem Außenschaft (20) zum Übertragen zumindest entweder einer Kraft oder eines Drehmoments zum Steuern der zweiten Wirkeinrichtung (50);
wobei der Außenschaft (20) zumindest entweder **gekrümmt** oder **krümmbar** ist oder ein Schwenkgelenk (24) **aufweist,**
wobei die erste Übertragungseinrichtung (40) und die zweite Übertragungseinrichtung (60) jeweils zumindest abschnittsweise **biegeflexibel** ausgebildet sind,
**dadurch gekennzeichnet, dass**
die zweite Übertragungseinrichtung (60) mittels einer **Bajonettkupplung** (63, 64) mit dem Werkzeug (50) lösbar mechanisch verbunden ist,
an der zweiten Übertragungseinrichtung (60) eine **Verriegelungseinrichtung** (47, 67; 46, 66) zur Kopplung der zweiten Übertragungseinrichtung (60) mit der ersten Übertragungseinrichtung (40) oder mit dem Außenschaft (20) derart, dass die zweite Übertragungseinrichtung (60) relativ zu der ersten Übertragungseinrichtung (40) oder relativ zu dem Außenschaft (20) nicht rotierbar ist, vorgesehen ist.

2. Medizinisches Instrument (10) nach dem vorangehenden Anspruch, bei dem Werkzeug (30, 50) und Außenschaft (20) **Kupplungseinrichtungen** (27, 37) zum lösbaren mechanischen Verbinden des Werkzeugs (30, 50) mit dem **distalen** Ende des Außenschafts (20) aufweisen.

3. Medizinisches Instrument (10) nach einem der vorangehenden Ansprüche, bei dem Außenschaft (20) und Handhabungseinrichtung (18) **Kupplungseinrichtungen** zum lösbaren mechanischen Verbinden des **proximalen** Endes (21) des Außenschafts (20) mit der Handhabungseinrichtung (18) aufweisen.

4. Medizinisches Instrument (10) nach einem der vorangehenden Ansprüche, bei dem die erste Übertragungseinrichtung (40) und die zweite Übertragungseinrichtung (60) **koaxial** im Außenschaft (20) angeordnet sind.

5. Medizinisches Instrument (10) nach einem der vorangehenden Ansprüche, bei dem die erste Übertragungseinrichtung (40) und die zweite Übertragungseinrichtung (60) **nebeneinander** im Außenschaft (20) angeordnet sind.

6. Medizinisches Instrument (10) nach einem der vorangehenden Ansprüche mit einem **Schwenkgelenk** (24), ferner mit:
einer dritten **Übertragungseinrichtung** (70), deren distales Ende mit dem **Schwenkgelenk** (24) gekoppelt ist, zum Steuern des Schwenkgelenks (24).

7. Medizinisches Instrument (10) nach dem vorangehenden Anspruch, ferner mit:
einem **Rotationsgelenk** proximal des Schwenkgelenks (24), zum Rotieren des Schwenkgelenks (24) relativ zum proximale Ende (21) des Außenschafts (20),
wobei die dritte Übertragungseinrichtung (70) ferner zum Steuern des Rotationsgelenks ausgebildet ist.

8. Medizinisches Instrument (10) nach dem vorangehenden Anspruch, bei dem die dritte Übertragungseinrichtung (70) **koaxial** in dem Außenschaft (20) angeordnet ist.

9. Medizinisches Instrument (10) nach einem der vorangehenden Ansprüche, bei dem die erste Wirkeinrichtung (30) als **bipolar-**elektrochirurgisches Instrument mit voneinander elektrisch isolierten Elektroden (32, 34) ausgebildet ist, wobei je eine der voneinander elektrisch isolierten Elektroden (32, 34) mit dem Außenschaft (20) und mit der ersten Übertragungseinrichtung (40) elektrisch leitfähig verbunden ist.

10. Medizinisches Instrument (10) nach einem der vorangehenden Ansprüche, bei dem an der zweiten Übertragungseinrichtung (60) eine **Verriegelungseinrichtung** (47, 67; 46, 66) zur Kopplung der zweiten Übertragungseinrichtung (60) mit einer dritten Übertragungseinrichtung (70) derart, dass die zweite Übertragungseinrichtung (60) relativ zu der dritten Übertragungseinrichtung (70) nicht rotierbar ist, vorgesehen ist.

11. Medizinisches Instrument (10) nach einem der vorangehenden Ansprüche, ferner mit:
einem **Rotationsgelenk** proximal des Werkzeugs (30, 50), zum Rotieren des Werkzeugs (30, 50) relativ zum Außenschaft (20).

## Claims

1. Medical instrument (10), with:
an outer shaft (20);
a maneuvering device (18) at the proximal end (21) of the outer shaft (20);
a tool (30, 50) at the distal end of the outer shaft (20), with a first working device (30) for a first function and a second working device (50) for a second function;
a first transmission device (40) in the outer shaft (20) for transmitting at least either a force or a torque for controlling the first working device (30);
a second transmission device (60) in the outer shaft (20) for transmitting at least either a force or a torque for controlling the second working device (50);
wherein the outer shaft (20) is at least either curved or able to be curved or has a pivot joint (24),
wherein the first transmission device (40) and the second transmission device (60) are each designed to be flexible at least in sections,
**characterized in that**
the second transmission device (60) is mechanically connected to the tool (50) in a releasable manner by means of a bayonet coupling (63, 64),
a locking device (47, 67; 46, 66) is provided on the second transmission device (60) for the purpose of coupling the second transmission device (60) to the first transmission device (40) or to the outer shaft (20), in such a way that the second transmission device (60) is not rotatable relative to the first transmission device (40) or relative to the outer shaft (20).

2. Medical instrument (10) according to the preceding claim, in which tool (30, 50) and outer shaft (20) have coupling devices (27, 37) for releasable mechanical connection of the tool (30, 50) to the distal end of the outer shaft (20).

3. Medical instrument (10) according to one of the preceding claims, in which outer shaft (20) and maneuvering device (18) have coupling devices for releasable mechanical connection of the proximal end (21) of the outer shaft (20) to the maneuvering device (18).

4. Medical instrument (10) according to one of the preceding claims, in which the first transmission device (40) and the second transmission device (60) are arranged coaxially in the outer shaft (20).

5. Medical instrument (10) according to one of the preceding claims, in which the first transmission device (40) and the second transmission device (60) are arranged alongside each other in the outer shaft (20).

6. Medical instrument (10) according to one of the preceding claims, with a pivot joint (24), and also with:
a third transmission device (70), of which the distal end is coupled to the pivot joint (24), and which controls the pivot joint (24).

7. Medical instrument (10) according to the preceding claim, also with:
a rotation joint arranged proximally of the pivot joint (24), for rotating the pivot joint (24) relative to the proximal end (21) of the outer shaft (20),
wherein the third transmission device (70) is further designed to control the rotation joint.

8. Medical instrument (10) according to the preceding claim, in which the third transmission device (70) is arranged coaxially in the outer shaft (20).

9. Medical instrument (10) according to one of the preceding claims, in which the first working device (30) is designed as a bipolar electrosurgical instrument with mutually electrically insulated electrodes (32, 34), wherein one of the mutually electrically insulated electrodes (32, 34) is in each case connected electrically conductively to the outer shaft (20) and to the first transmission device (40).

10. Medical instrument (10) according to one of the preceding claims, in which a locking device (47, 67; 46, 66) is provided on the second transmission device (60) for the purpose of coupling the second transmission device (60) to a third transmission device (70), in such a way that the second transmission device (60) is not rotatable relative to the third transmission device (70).

11. Medical instrument (10) according to one of the preceding claims, also with:
a rotation joint arranged proximally of the tool (30, 50), for rotating the tool (30, 50) relative to the outer shaft (20).

## Revendications

1. Instrument médical (10), avec
une tige extérieure (20);
un dispositif de manipulation (18) à l'extrémité proximale (21) de la tige extérieure (20);
un outil (30, 50) à l'extrémité distale de la tige extérieure (20), avec un premier dispositif actif (30) pour une première fonction et un deuxième dispositif actif (50) pour une deuxième fonction;
un premier dispositif de transmission (40) dans la tige extérieure (20) pour la transmission d'au moins soit une force soit un couple pour la commande du premier dispositif actif (30);
un deuxième dispositif de transmission (60) dans la tige extérieure (20) pour la transmission d'au moins soit une force soit un couple pour la commande du deuxième dispositif actif (50);
la tige extérieure (20) soit étant courbée ou pouvant être courbée soit présentant une articulation pivotante (24),
le premier dispositif de transmission (40) et le deuxième dispositif de transmission (60) étant constitués respectivement au moins par tronçons de façon souple en flexion,
**caractérisé en ce que**
le deuxième dispositif de transmission (60) est raccordé mécaniquement de façon détachable à l'outil (50) au moyen d'un accouplement à baïonnette (63, 64),
un dispositif de verrouillage (47, 67; 46, 66) destiné à l'accouplement du deuxième dispositif de transmission (60) avec le premier dispositif de transmission (40) ou avec la tige extérieure (20) est prévu sur le deuxième dispositif de transmission (60) de telle sorte que le deuxième dispositif de transmission (60) ne peut pas tourner par rapport au premier dispositif de transmission (40) ou par rapport à la tige extérieure (20).

2. Instrument médical (10) selon la revendication précédente, dans lequel l'outil (30, 50) et la tige extérieure (20) présentent des dispositifs d'accouplement (27, 37) pour le raccordement mécanique détachable de l'outil (30, 50) à l'extrémité distale de la tige extérieure (20).

3. Instrument médical (10) selon l'une des revendications précédentes, dans lequel la tige extérieure (20) et le dispositif de manipulation (18) présentent des dispositifs d'accouplement pour le raccordement mécanique détachable de l'extrémité proximale (21) de la tige extérieure (20) au dispositif de manipulation (18).

4. Instrument médical (10) selon l'une des revendications précédentes, dans lequel le premier dispositif de transmission (40) et le deuxième dispositif de transmission (60) sont disposés de façon coaxiale dans la tige extérieure (20).

5. Instrument médical (10) selon l'une des revendications précédentes, dans lequel le premier dispositif de transmission (40) et le deuxième dispositif de transmission (60) sont disposés l'un à côté de l'autre dans la tige extérieure (20).

6. Instrument médical (10) selon l'une des revendications précédentes, avec une articulation pivotante (24), avec en plus :
un troisième dispositif de transmission (70) dont l'extrémité distale est couplée à l'articulation pivotante (24) pour la commande de l'articulation pivotante (24).

7. Instrument médical (10) selon la revendication précédente, avec en plus:
une articulation rotative proximale relativement à l'articulation pivotante (24), pour la rotation de l'articulation pivotante (24) par rapport à l'extrémité proximale (21) de la tige extérieure (20),
le troisième dispositif de transmission (70) étant en outre constitué pour la commande de l'articulation rotative.

8. Instrument médical (10) selon la revendication précédente, dans lequel le troisième dispositif de transmission (70) est disposé de façon coaxiale dans la tige extérieure (20).

9. Instrument médical (10) selon l'une des revendications précédentes, dans lequel le premier dispositif actif (30) est constitué en tant qu'instrument électrochirurgical bipolaire avec des électrodes (32, 34) isolées électriquement les unes des autres, une des électrodes (32, 34) isolées électriquement les unes des autres étant raccordée respectivement de façon électriquement conductrice à la tige extérieure (20) et au premier dispositif de transmission (40).

10. Instrument médical (10) selon l'une des revendications précédentes, dans lequel il est prévu sur le deuxième dispositif de transmission (60) un dispositif de verrouillage (47, 67; 46, 66) pour l'accouplement du deuxième dispositif de transmission (60) avec un troisième dispositif de transmission (70) de telle sorte que le deuxième dispositif de transmission (60) ne peut pas tourner par rapport au troisième dispositif de transmission (70).

11. Instrument médical (10) selon l'une des revendications précédentes, avec en plus:
une articulation rotative proximale relativement à l'outil (30, 50) pour faire tourner l'outil (30, 50) par rapport à la tige extérieure (20).
